# FASCICULE DE BREVET EUROPEEN

(11) **EP 2 083 775 B1**
(45) Date de publication et mention de la délivrance du brevet: **08.06.2011**
(21) Numéro de dépôt: 07858406.7
(22) Date de dépôt: 08.10.2007
(51) Int. Cl.: A61F 9/007

(54) **BOUCHON MEATIQUE A POSE SIMPLIFIEE**
LEICHT ANZUWENDENDER MEATUSSTÖPSEL
EASY-APPLICATION MEATAL PLUG

(30) Priorité: 09.10.2006 FR 0608829
(43) Date de publication de la demande: 05.08.2009
(73) Titulaire: France Chirurgie Instrumentation SAS-FCI, 75015 Paris (FR)
(72) Inventeur: BERNARD, Pascal, 17737 Nieul Sur Mer (FR)
(74) Mandataire: Eidelsberg, Olivier Nathan
(86) Numéro de dépôt international: PCT/FR2007/001637
(87) Numéro de publication internationale: WO 2008/043905

(56) Documents cités:
- WO-A-03/057101
- JP-A- 2005 000 628
- US-A- 5 163 959

## Description

La présente invention se rapporte à un bouchon méatique destiné à boucher une ouverture méatique d'un conduit lacrymal de l'oeil humain. Un dispositif de pose d'un bouchon de ce genre et un procédé pour boucher une ouverture méatique d'un conduit lacrymal d'un oeil de l'être humain sont aussi décrits.

Les bouchons méatiques connus aujourd'hui sont constitués d'un corps ayant une tige et un bulbe disposé à une extrémité de la tige et faisant saillie latéralement de celle-ci à la manière d'un champignon. Ce bulbe est inséré dans le conduit lacrymal suffisamment profondément pour atteindre un épaulement situé au-delà du conduit et ainsi, en butant contre l'épaulement du conduit lacrymal, se bloquer pour empêcher le bouchon de s'échapper du conduit. Ce type de dispositifs fonctionne très bien. Cependant, il est difficile à poser et notamment à insérer dans le conduit lacrymal. Pour ce faire, il faut dilater le conduit pour permettre le passage du bulbe du bouchon. Le document JP-A-2005000628 décrit un bouchon cylindrique comprenant les caractéristiques du préambule de la revendication 1.

La présente invention vise à surmonter ces inconvénients en proposant un bouchon méatique que l'on peut poser plus simplement et plus rapidement dans le conduit lacrymal et notamment qui ne nécessite pas une dilatation préalable du conduit lacrymal ou du moins ne nécessite qu'une moindre dilatation de celui-ci.

Suivant l'invention, un bouchon méatique est tel que défini à la revendication 1.

Les inventeurs se sont rendus compte pour la première fois qu'en formant ainsi un bulbe ayant une forme aplatie et creuse, on pouvait facilement, en utilisant un pousseur étireur que l'on insère par l'intermédiaire du trou, préétirer le bouchon dans la direction de l'axe de la tige pour diminuer temporairement l'épaisseur du bulbe et permettre son insertion dans le conduit lacrymal. Dans le même temps, le fait de prévoir un bulbe de forme aplatie ou ramassée permet de s'assurer que le bouchon résiste à l'opération d'allongement étirage et reprend une forme après retrait du dispositif de pose qui lui permet de bien "tenir" dans le conduit lacrymal, notamment au niveau de l'épaulement au-delà du conduit.

Le trou se termine dans le bulbe en s'évasant, pour former une chambre creuse ayant une dimension en largeur supérieure à la dimension en largeur du trou au niveau de la tige, de préférence une dimension en largeur supérieure à la dimension en largeur de la tige elle-même.

De préférence, il est prévu à l'extrémité de la tige opposée au bulbe, une collerette faisant saillie au-delà de la tige latéralement.

De préférence, la collerette a une forme cylindrique de révolution par rapport à l'axe de la tige.

De préférence, le bulbe a une forme telle que sa surface extérieure est arrondie, sans comporter de coins en forme d'angle.

La déformation du bouchon avant son insertion dans le canal est ainsi facilitée.

De préférence, la tige est de forme cylindrique circulaire.

Un dispositif pour insérer un bouchon suivant l'invention est aussi décrit.

Un dispositif suivant l'invention comportant un corps formant poussoir, en forme de tige, destiné à être inséré dans le trou formé dans un bouchon méatique pour l'étirer, est caractérisé en ce qu'il est prévu des moyens de retenue destinés à retenir le bouchon en un point de retenue pendant que la tige y est insérée, pour étirer le bouchon par rapport au point de retenue.

Les moyens de retenue sont constitués de deux pinces disposées à l'extrémité du dispositif par laquelle la tige est poussée, les au moins deux pinces pouvant prendre une première position dans laquelle elles forment conjointement une butée en forme de plaque percée d'un trou par lequel peut passer la tige d'un bouchon mais par lequel ne peut pas passer une collerette lorsque celle-ci est prévue à une extrémité de la tige du bouchon, et une deuxième position écartées l'une de l'autre, dans laquelle le bouchon, et notamment la collerette, est libéré des deux pinces.

Ainsi, pour insérer le bouchon on fait en sorte que la collerette vienne buter contre la plaque formée par l'extrémité des deux pinces pour ensuite pousser la tige à travers le trou formé dans le bouchon pour étirer le bouchon et le disposer dans le canal lacrymal, puis on retire la tige et on écarte les pinces pour libérer le bouchon qui est ainsi placé en position.

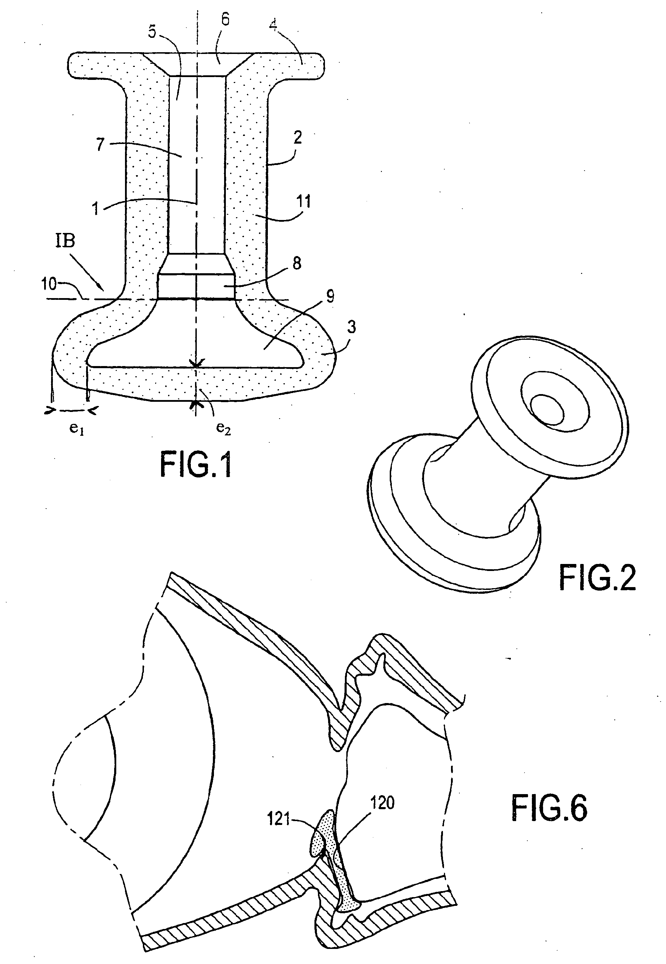

On décrit maintenant à titre d'exemple, en se reportant au dessin, un mode de réalisation de l'invention.
A la figure 1 il est représenté une vue en coupe longitudinale d'un bouchon suivant l'invention ;
A la figure 2 il est représenté une vue en perspective d'un bouchon suivant l'invention ;
la figure 3 est une vue en perspective éclatée d'un dispositif de pose et d'étirage suivant l'invention ; et
la figure 4 est une vue en perspective à plus grande échelle d'une partie d'extrémité du dispositif de la figure 3, tandis que la figure 5 est une vue en coupe d'une partie d'extrémité du dispositif de la figure 4 ; et
la figure 6 est une vue en coupe d'une partie de l'oeil d'un être humain comportant un bouchon disposé suivant l'invention.

A la figure 1 il est représenté une vue en coupe longitudinale d'un bouchon méatique suivant l'invention. Ce bouchon, en matière extensible et étirable, notamment en silicone, notamment revêtu d'une fine couche de Poly Vinyl Pyrrolidone, est de forme cylindrique de révolution par rapport à l'axe 1 longitudinal. Le bouchon méatique comporte une tige 2 de forme cylindrique circulaire prolongée à une extrémité par un bulbe 3 faisant saillie latéralement de la tige 2. A l'autre extrémité de la tige 2 est formée une collerette 4 qui est ici formée sensiblement perpendiculairement à l'axe 1 longitudinal. Suivant un autre mode de réalisation, on pourrait également prévoir une collerette inclinée par rapport à l'axe 1 longitudinal d'un angle différent de 90°. Un trou 5 est formé dans le bouchon. Il débouche à une ouverture 6 du côté de la collerette 4. Ce trou 5 est constitué d'un premier tronçon 7 qui s'étend sensiblement sur toute la longueur de la tige 2 pour ensuite s'évaser dans un deuxième tronçon 8 intermédiaire et se terminer par une chambre 9 à l'intérieur du bulbe 3. La plus grande dimension en largeur (dans la direction perpendiculaire à l'axe 1) de la chambre 9 est supérieure à la dimension en largeur du tronçon 7 et également, de préférence, supérieure à la dimension en largeur de la tige 2.

On définit suivant l'invention une ligne ou plan de départage entre le bulbe et la tige comme étant la droite 10 qui sépare la tige 2 et le bulbe 3. Cette droite 10 se trouve à l'endroit où se termine la tige 2, c'est-à-dire à l'endroit où sa surface extérieure commence à faire saillie au-delà de la paroi 11 sensiblement verticale. En dessous de la droite 10 de délimitation, il y a la tige et de l'autre côté il y a le bulbe.

Suivant un mode de réalisation préféré de l'invention, le trou 5 a un diamètre de 0,33 mm dans le tronçon 7, tandis que la tige 2 a un diamètre de 0,55 mm.

La plus grande dimension en largeur de la chambre 9 est sensiblement égale à 0,85 mm. La plus grande dimension en largeur du bulbe 3 est sensiblement égale à 1,1 mm. La dimension suivant l'axe longitudinal du bulbe 3, c'est-à-dire la dimension comprise entre la droite 10 et le sommet du bulbe 3, est de 0,38 mm. La dimension en longueur de la tige 2 à partir de l'ouverture 6 jusqu'au plan 10 est de 1 mm. La dimension en longueur (dans la direction de l'axe 1) de la collerette est d'environ 0,1 mm. La dimension la plus grande en largeur de la collerette est d'environ 1 mm.

Le rapport de la dimension en longueur (hauteur) de la tige 2 sur le rapport de la dimension en longueur (hauteur) du bulbe 3 est ainsi égal à 1/0,38, soit 2,6.

Le rapport de la dimension en largeur du bulbe 3 avec sa dimension en hauteur est de 1,1/0,38, soit 2,9.

La paroi délimitant la cavité formée dans le bulbe a une épaisseur d'environ 0,12 mm. La surface extérieure du bulbe 3 est arrondie, notamment en étant constituée en section transversale longitudinale (figure 1) d'une succession d'arcs de cercle. Elle ne comporte pas de coins formant un angle. L'épaisseur de la paroi délimitant le tronçon 7 de la tige 2 est de 0,18 mm.

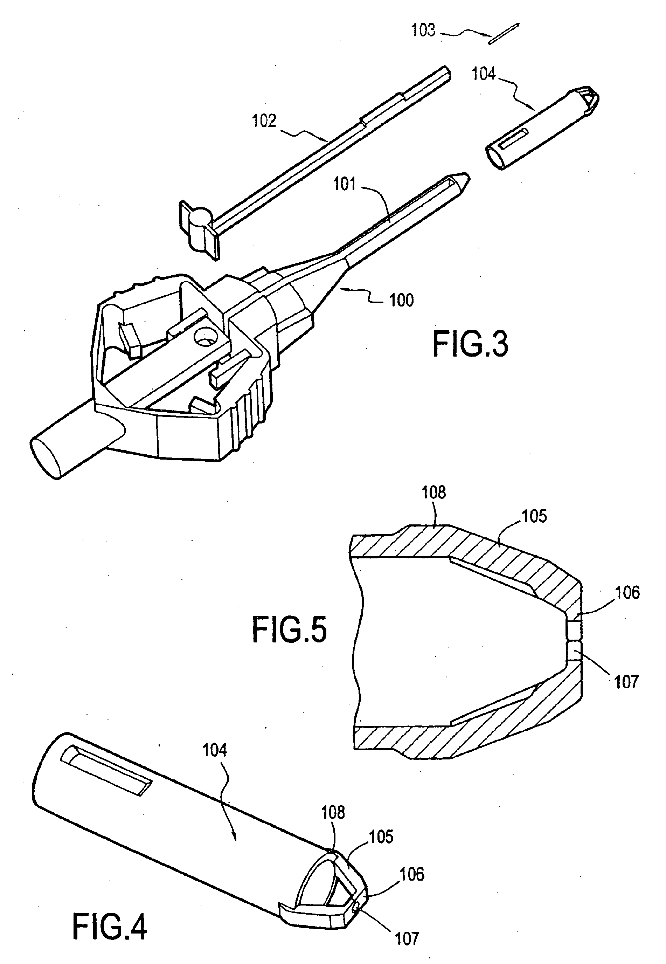

A la figure 3 il est représenté un dispositif destiné à insérer en position un bouchon suivant l'invention. Ce dispositif est constitué d'un corps principal 100 dans lequel est défini un conduit 101 par lequel peut être poussée une tige 102. La tige 102 supporte à son extrémité une aiguille 103 de poussée destinée à s'insérer dans le trou 5 du bouchon pour venir jusque dans la cavité 9, pousser le fond de la cavité 9 et étirer la paroi du bulbe du bouchon 9 jusqu'à la déformer et la rendre moins aplatie pour permettre son insertion dans le canal 104 lacrymal. A l'extrémité du dispositif 100, du côté où va être poussée la tige 102, il est prévu un manchon 104 qui peut être amovible par rapport au corps 100.

Ce manchon 104 amovible, de forme cylindrique circulaire creux comporte à son extrémité, destinée à se trouver du côté où va être poussée l'extrémité 103 de la tige 102, deux pinces 105 qui, à leur extrémité libre, viennent en contact l'une avec l'autre pour former une plaque 106 de butée à travers laquelle est formé un trou 107 dont le diamètre est tel qu'il peut enserrer la tige 2 sans que la collerette 4 puisse, elle, passer à travers. Ces deux pinces 105 sont reliées au manchon 104 par deux zones 108 de faible épaisseur qui permettent ainsi facilement d'écarter les deux pinces 105 l'une de l'autre pour ainsi permettre de libérer le bouchon.

Pour insérer le bouchon dans le canal 120 lacrymal, on dispose les pinces 105 pour que le trou 107 vienne encercler la tige 2 tandis que la collerette 4 vient buter contre la plaque 106. Ensuite, on pousse la tige 102 en la faisant passer dans le manchon 104 creux et dans le trou 107 pour venir dans le trou 5, pour venir pousser et étirer le bulbe 3. Une fois le bulbe 3 étiré et ainsi rendu moins aplati, on peut le pousser dans le canal 120 jusqu'à ce que le bulbe 3 dépasse de l'épaulement 121 qui est formé à l'extrémité du canal lacrymal. Une fois dans cette position, on retire la tige et on écarte les pinces l'une de l'autre pour libérer le bouchon méatique.

A la figure 6, on a représenté un bouchon suivant un mode de réalisation dans lequel la collerette est inclinée d'un angle différent de 90° par rapport à la tige, notamment de 70° à 80°, ce qui permet d'avoir une meilleure conformité avec l'ouverture du côté de l'oeil du canal lacrymal.

Le matériau utilisé pour le bouchon est notamment en silicone PDS ou poly diméthyl siloxane, notamment d'une dureté de 30 à 80 Shore, par exemple 50 Shore.

Le matériau et les épaisseurs du bouchon, notamment la ou les épaisseurs au niveau du bulbe, sont choisis pour que le bulbe ait une élasticité suffisante pour reprendre sensiblement sa forme après avoir été étiré dans la direction de l'axe longitudinal de la tige et en même temps soit suffisamment résistant pour supporter cet étirement (c'est-à-dire un étirement tel que à l'état étiré le bouchon ait une largeur sensiblement égale à celle de la tige) sans être endommagé.

Par exemple pour du silicone PDS à 50 Shore, l'épaisseur e₁ de la paroi du bulbe au niveau de sa plus grande largeur est de préférence comprise entre 0,14 mm et 0,18 mm, notamment est égale à 0,16 mm, tandis que l'épaisseur e₂ au niveau de la section avec l'axe longitudinale est de préférence comprise entre 0,10 mm et 0,20 mm, notamment est égale à 0,12 mm.

Le rapport de la largeur du bulbe sur sa hauteur est comprise entre 1 et 5, de préférence entre 2 et 4.

## Revendications

1. Bouchon méatique de forme cylindrique par rapport à un axe longitudinal (1), notamment cylindrique circulaire, comportant une tige (2) et un bulbe (3) disposée à une extrémité de la tige, le bulbe faisant saillie latéralement au-delà de la tige, un trou (5, 6, 7, 9) étant formé dans le bouchon, le trou débouchant à l'extrémité de la tige opposée au bulbe, et le trou s'étendant au moins jusque dans le bulbe, le rapport de la dimension en hauteur de la tige, mesurée le long de l'axe longitudinal (1), à la dimension en hauteur suivant ce même axe du bulbe, est supérieur à 1, de préférence supérieur à 2,5 ; et le rapport de la dimension en largeur, c'est-à-dire mesurée dans la direction perpendiculaire à l'axe (1), du bulbe sur sa hauteur est supérieure à 1, de préférence supérieure à 2,5, **caractérisé en ce que** le trou se termine dans le bulbe en s'évasant dans la direction à l'opposé de la tige, pour former une chambre (9) creuse ayant une dimension en largeur supérieure à la dimension en largeur du trou au niveau de la tige.

2. Bouchon suivant la revendication 1, **caractérisé en ce qu'**il est prévu à l'extrémité de la tige opposée au bulbe, une collerette (4) faisant saillie au-delà de la tige latéralement.

3. Bouchon suivant la revendication 1 ou 2, **caractérisé en ce qu'**une dimension du trou en largeur est supérieure à la dimension en largeur de la tige elle-même.

4. Bouchon suivant l'une des revendications 1 à 3, **caractérisé en ce que** le bulbe a une forme telle que sa surface extérieure est arrondie, sans comporter de coins en forme d'angle, la déformation du bouchon avant son insertion dans le canal étant ainsi facilitée.

5. Bouchon suivant l'une des revendications 1 à 4, **caractérisé en ce que** le bouchon est en silicone, notamment PDS.

6. Bouchon suivant l'une des revendications 1 à 5, **caractérisé en ce** l'épaisseur e₁ de la paroi du bulbe au niveau de sa plus grande largeur est de préférence comprise entre 0,14 mm et 0,18 mm, notamment est égale à 0,16 mm, tandis que l'épaisseur e₂ au niveau de la section avec l'axe longitudinale est de préférence comprise entre 0, 10 mm et 0,20 mm, notamment est égale à 0, 12 mm.

7. Bouchon suivant l'une des revendications 1 à 6, **caractérisé en ce que** le rapport de la largeur du bulbe sur sa hauteur est comprise entre 1 et 5, de préférence entre 2 et 4.

## Claims

1. Meatal plug of cylindrical shape, in particular circular cylindrical, in relation to a longitudinal axis (1), comprising a stem (2) and a bulb (3) arranged at one end of the stem, the bulb protruding laterally over said stem, a hole (5, 6, 7, 9) being formed in the plug, said hole opening at the end of the stem opposite the bulb and extending at least into the bulb, the ratio of the height dimension of the stem, measured along the longitudinal axis (1), to the height dimension along this same bulb axis, being greater than 1, preferably greater than 2.5; and the ratio of the width dimension of the bulb to its height, i.e. measured in a direction perpendicular to the axis (1), being greater than 1, preferably greater than 2.5, **characterised in that** the hole terminates in the bulb by flaring out in the direction opposite to the stem to form a hollow chamber (9) with a width dimension greater than the width dimension of the hole at the level of the stem.

2. Plug according to claim 1, **characterised in that** at the end of the stem opposite the bulb a collar (4) is provided which projects over the stem laterally.

3. Plug according to one of claims 1 or 2, **characterised in that** a width dimension of the hole is greater than the width dimension of the stem itself.

4. Plug according to one of claims 1 to 3, **characterised in that** the bulb is shaped such that its outer surface is rounded, without any angled corners, the deformation of the plug prior to its insertion into the canal thus being facilitated.

5. Plug according to one of claims 1 to 4, **characterised in that** the plug is made of silicone, in particular PDMS.

6. Plug according to one of claims 1 to 5, **characterised in that** the thickness e₁ of the wall of the bulb at its greatest width is preferably between 0.14 mm and 0.18 mm, in particular 0.16 mm, whereas the thickness e₂ at the section with the longitudinal axis is preferably between 0.10 mm and 0.20 mm, in particular 0.12 mm.

7. Plug according to one of claims 1 to 6, **characterised in that** the ratio of the width of the bulb to its height is between 1 and 5 , preferably between 2 and 4.

## Patentansprüche

1. Meatusstöpsel mit einer zur Längsachse (1) zylindrischen, insbesondere kreiszylindrischen Form, der einen Schaft (2) und eine Wulst (3) aufweist, die an einem Ende des Schafts angeordnet ist, wobei die Wulst seitwärts über den Schaft hinausragt, wobei in dem Stöpsel ein Loch (5, 6, 7, 9) geformt ist, wobei das Loch zu dem der Wulst gegenüberliegendem Ende des Schaftes hin mündet und sich das Loch mindestens bis in die Wulst erstreckt, wobei das Verhältnis von der Abmessung der Höhe des Schafts, gemessen entlang der Längsachse (1), zu der Abmessung der Höhe der Wulst entlang derselben Achse größer als 1 und vorzugsweise größer als 2,5 ist; und das Verhältnis von der Abmessung der Breite der Wulst, das heißt in der Richtung senkrecht zur Achse (1) gemessen, zu der Abmessung von deren Höhe größer als 1 und vorzugsweise größer als 2,5 ist,
**dadurch gekennzeichnet, dass** das Loch sich in der dem Schaft gegenüberliegenden Richtung erweiternd in der Wulst endet, um eine hohle Kammer (9) zu bilden, deren Abmessung der Breite größer ist als die Abmessung der Breite des Lochs im Bereich des Schaftes.

2. Stöpsel nach Anspruch 1, **dadurch gekennzeichnet, dass** am Ende des Schafts gegenüber der Wulst ein Kragen (4) vorgesehen ist, der seitwärts über den Schaft hinausragt.

3. Stöpsel nach Anspruch 1 oder 2, **dadurch gekennzeichnet, dass** eine Abmessung der Breite des Lochs größer ist als die Abmessung der Breite des Schafts selbst.

4. Stöpsel nach einem der Ansprüche 1 bis 3, **dadurch gekennzeichnet, dass** die Wulst derart geformt ist, dass ihre Außenfläche abgerundet ist, ohne winklig geformte Ecken aufzuweisen, sodass die Verformung des Stöpsels vor dem Einführen in den Kanal vereinfacht ist.

5. Stöpsel nach einem der Ansprüche 1 bis 4, **dadurch gekennzeichnet, dass** der Stöpsel aus Silikon besteht, insbesondere Polydimethylsiloxan.

6. Stöpsel nach einem der Ansprüche 1 bis 5, **dadurch gekennzeichnet, dass** die Dicke e₁ der Wand der Wulst im Bereich ihrer größten Breite vorzugsweise zwischen 0,14 mm und 0,18 mm beträgt, insbesondere 0,16 mm beträgt, während die Dicke e₂ im Schnittbereich mit der Längsachse vorzugsweise zwischen 0,10 mm und 0,20 mm beträgt, insbesondere 0,12 mm beträgt.

7. Stöpsel nach einem der Ansprüche 1 bis 6, **dadurch gekennzeichnet, dass** das Verhältnis von der Breite der Wulst zu ihrer Höhe zwischen 1 und 5, vorzugsweise zwischen 2 und 4 beträgt.
